# FASCICULE DE BREVET EUROPEEN

(11) **EP 0 755 675 B1**
(45) Date de publication et mention de la délivrance du brevet: **17.09.1997**
(21) Numéro de dépôt: 96401491.4
(22) Date de dépôt: 05.07.1996
(51) Int. Cl.: A61K 7/48, A61K 47/24, A61K 47/34

(54) **Composition dermatologique ou pharmaceutique, procédé de préparation et utilisation**
Dermatologische und/oder pharmazeutische Zubereitung, ihre Herstellung und Anwendung
Dermatological and/or pharmaceutical composition, process of preparation and utilization

(30) Priorité: 28.07.1995 FR 9509252
(43) Date de publication de la demande: 29.01.1997
(73) Titulaire: L'OREAL, 75008 Paris (FR)
(72) Inventeur: Grollier, Jean-François, 75006 Paris (FR); Allec, Josiane, Les Vergères de Val Constance, 06600 Antibes (FR); Agostini, Isabelle, 92290 Chatenay Malabry (FR)
(74) Mandataire: Dodin, Catherine

(56) Documents cités:
- EP-A- 0 150 914
- EP-A- 0 197 485
- EP-A- 0 336 902
- EP-A- 0 602 905
- CHEMICAL ABSTRACTS, vol. 119, no. 22, 29 Novembre 1993 Columbus, Ohio, US; abstract no. 233712z, page 537; XP002016646 & JP-A-05 201 851 (TOSHIBA SILICONE) 10 Août 1993
- PATENT ABSTRACTS OF JAPAN vol. 10, no. 230 (C-365) [2286] , 9 Août 1986 & JP-A-61 065808 (SHISEIDO CO LTD), 4 Avril 1986,

## Description

La présente invention a trait à une composition dermatologique ou pharmaceutique à application topique, susceptible d'être appliquée sur la peau ou les muqueuses, en particulier sur les lèvres, à titre préventif et/ou curatif.

On sait que des lésions cutanées ou des muqueuses, notamment des lèvres, peuvent être générées par une infection due à des micro-organismes, et en particulier de type virale, bactérienne ou fongique, par des phénomènes inflammatoires immunodépendants ou non, par des phénomènes de type neurogénique, par des dysfonctionnements cellulaires tels que les désordres de la prolifération, de la différenciation ou de la pigmentation, et/ou par des facteurs externes tels que le froid, les UV, les brûlures, l'absorption orale de certains médicaments, les allergènes ou irritants, ou les piqûres d'insectes.

On connaît diverses compositions dermatologiques ou pharmaceutiques susceptibles de prévenir de telles lésions et/ou de traiter la zone atteinte, ainsi que les zones adjacentes, après apparition de ces lésions.
Ces compositions peuvent notamment se présenter sous forme de produits pâteux tels que des onguents, des crèmes ou des gels, sous forme de sticks ou bien sous forme de solutions à appliquer localement.

Toutefois, on a constaté que lorsque ces compositions sont appliquées sur la peau, elles présentent l'inconvénient de transférer. On entend par là qu'elles peuvent se déposer au moins en partie, en laissant une trace, sur des supports avec lesquels elles seraient en contact, tels que, en particulier un verre, un vêtement ou la peau.
Il s'en suit, premièrement, une persistance médiocre et un temps de contact limité de ladite composition dermatologique ou pharmaceutique sur la zone de peau à traiter.
Il s'en suit deuxièmement un dépôt peu plaisant sur le support, en particulier lorsqu'il s'agit d'une tierce personne, avec un effet de tachage éventuel dudit support.
Il s'en suit également un risque de contamination, pour le support, par les agents actifs et/ou par les agents infectieux présents sur la peau de la personne à traiter.

Il est donc ainsi nécessaire de renouveler régulièrement l'application de la composition sur la peau ou les muqueuses, tout en évitant le contact avec tout support de manière à diminuer les risques de transfert.
Un autre inconvénient des compositions de l'art antérieur réside dans leur tendance à migrer, c'est-à-dire dans le fait qu'elles ont tendance à se propager vers des zones adjacentes à la zone à traiter, notamment dans les ridules de la peau, diffusant les agents actifs et/ou infectieux.

La présente invention a pour but de pallier ou de limiter ces inconvénients et propose une composition qui permet d'obtenir un film de très bonne tenue, qui transfère peu ou pas du tout, et qui ne migre peu, voire pas du tout.

Ainsi, un objet de l'invention est une composition dermatologique ou pharmaceutique à application topique, comprenant une huile volatile, une huile siliconée phénylée et au moins un agent actif dermatologiquement et/ou pharmaceutiquement.

Un autre objet de l'invention est un procédé de préparation de ladite composition dans lequel on prépare tout d'abord un prémélange comprenant au moins une partie des différents constituants de la composition, dont les cires éventuelles; on chauffe ce prémélange en le malaxant, à une température à laquelle il fond; on ajoute à une température adéquate, pouvant être de l'ordre de la température ambiante, le reste des constituants tout en continuant le malaxage; puis on malaxe le mélange obtenu pendant au moins une partie de son refroidissement jusqu'à température ambiante s'il était à une température supérieure, caractérisé par le fait que l'opération de malaxage est effectuée au moins partiellement dans un extrudeur.

Encore un autre objet de l'invention est l'utilisation de l'association d'une huile volatile et d'une huile siliconée phénylée, dans une composition dermatologique ou pharmaceutique à application topique comprenant au moins un agent actif dermatologiquement et/ou pharmaceutiquement, dans le but d'améliorer le transfert et/ou la migration de ladite composition.

On a constaté que la composition selon l'invention permet l'obtention d'un film homogène, facilement applicable et s'étalant facilement et uniformément. Le film obtenu présente également une texture légère et reste confortable à porter tout au long de la journée.
Il favorise un temps de contact prolongé de l'actif avec la zone à traiter, et peut favoriser la pénétration de certains médicaments.
De plus, la présence sur la zone à traiter de la composition selon l'invention peut permettre de maintenir une humidification réduite, propice à un traitement accéléré de l'éventuelle lésion.
La composition selon l'invention peut, en outre, être avantageusement utilisée pour formuler des agents actifs instables en milieu aqueux.

La composition selon l'invention comprend donc une huile volatile, qui peut être choisie en particulier parmi les huiles hydrocarbonées ou les huiles siliconées, cycliques ou linéaires, seules ou en mélange.
Par huile volatile, on entend dans la présente description, toute huile susceptible de s'évaporer au contact de la peau. De préférence, on utilise des huiles dont le point éclair est suffisamment élevé pour permettre l'utilisation de ces huiles en formulation, et suffisamment bas pour obtenir l'effet évanescent souhaité. On emploie de préférence des huiles dont le point éclair est de l'ordre de 40-100°C. Parmi les huiles siliconées volatiles, on peut citer la cyclotétradiméthylsiloxane, la cyclopentadiméthylsiloxane, la cyclohexadiméthylsiloxane et le méthylhexyldiméthylsiloxane. Parmi les huiles hydrocarbonées volatiles, on peut citer les isoparaffines.
La composition selon l'invention peut comprendre de 8 à 80% en poids, de préférence de 30 à 60% en poids, d'huiles volatiles par rapport au poids total de la composition.

La composition selon l'invention comprend également une huile siliconée phénylée.
Cette huile peut être un polyphénylméthylsiloxane ou un phényltriméthicone, ou un mélange de différentes huiles siliconées phénylées, et en particulier peut répondre à la formule suivante : 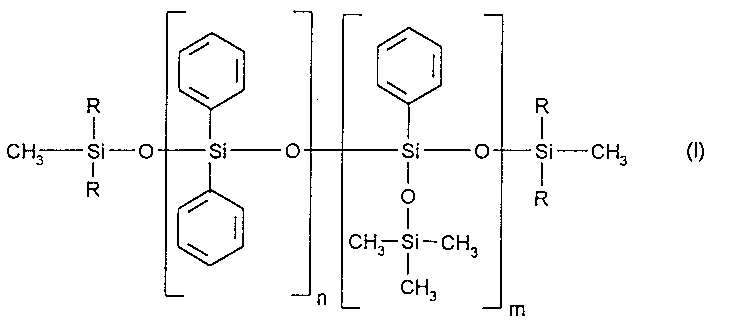 dans laquelle
. R est un radical alkyle en C1-C30, un radical aryle ou un radical aralkyle,
. n est un nombre entier compris entre 0 et 100,
. m est un nombre entier compris entre 0 et 100, sous réserve que la somme m+n est comprise entre 1 et 100.
De préférence, R est un radical méthyle, éthyle, propyle, isopropyle, décyle, dodécyle ou octadécyle, ou encore un radical phényle, tolyle, benzyle ou phénéthyle.
Parmi ces huiles phénylées, on peut citer l'huile Belsil PDM1000 de Wacker, les huiles DC556 ou SF558 de Dow Corning, l'huile Abil AV8853 de Goldschmidt ou l'huile Silbione 70633V30 de Rhône Poulenc.
La composition selon l'invention peut comprendre 1 à 35% en poids, de préférence 20 à 30% en poids, d'huiles siliconées phénylées.

La phase grasse peut comprendre, en plus des huiles ci-dessus, les corps gras usuellement utilisés dans le domaine considéré. Parmi ceux-ci, on peut citer les corps gras siliconés tels que les huiles, les gommes et les cires de silicone, ainsi que les corps gras non siliconés tels que les huiles ou les cires végétales, minérales, animales et/ou synthétiques, et leurs mélanges. On utilise de préférence des corps gras occlusifs ou filmogènes qui peuvent favoriser la pénétration de l'agent actif et l'effet'écran' de la composition.

Parmi les corps gras siliconés, on peut citer les polydiméthylsiloxanes (PDMS) et les alkyldiméthicones, ainsi que les silicones modifiées par des groupements aliphatiques et/ou aromatiques, éventuellement fluorés, ou par des groupements fonctionnels tels que des groupements hydroxyles, thiols et/ou amines.
Parmi les corps gras non siliconés, on peut citer l'huile de paraffine, de vaseline, le perhydrosqualène, l'huile d'arara, d'amande douce, de calophyllum, de palme, de ricin, d'avocat, de jojoba, d'olive ou de germes de céréales; des esters d'acides gras; des alcools; des acétylglycérides; des octanoates, décanoates ou ricinoléates d'alcools ou de polyalcools; des triglycérides d'acides gras; des glycérides; des huiles hydrogénées concrètes à 25°C; des lanolines; des esters gras concrets à 25°C; la cire d'abeilles; les cires végétales telles que la cire de Carnauba, de Candellila, d'Ourrury, du Japon ou les cires de fibres de lièges ou de canne à sucre; les cires minérales, par exemple de paraffine, de lignite ou les cires microcristallines ou les ozokérites; les cires synthétiques, parmi lesquelles les cires de polyéthylène et les cires obtenues par synthèse de Fischer-Tropsch.

Ces corps gras peuvent en particulier être choisis de manière variée par l'homme du métier afin de préparer une composition ayant les propriétés souhaitées, par exemple en consistance ou en texture.
En particulier, la composition selon l'invention peut comprendre au moins une cire, de manière à assurer la résistance mécanique, lorsque la composition se présente sous la forme d'un stick. Lorsqu'elle se présente sous la forme d'une pâte souple ou d'un produit coulé obtenu par un procédé classique, la composition selon l'invention comprend une quantité peu importante de cire, par exemple de l'ordre de 5-12% en poids.
Lorsqu'elle est obtenue par extrusion, la composition peut comprendre une quantité plus important de cires, et en particulier de préférence 12-60% en poids de cires ayant un point de fusion supérieur à 55°C.
D'une manière générale, la composition peut comprendre 0,5-30% en poids d'au moins une cire hydrocarbonée et/ou siliconée, et de préférence 10-20% en poids de cire hydrocarbonée et 0-10% en poids de cire siliconée.

La composition selon l'invention comprend également au moins un agent actif, parmi lesquels on peut citer les agents actifs contre les micro-organismes, notamment à activité antivirale, antibactérienne ou antifongique; les agents à activité anti-inflammatoire ou immunomodulatrice; les agents antagonistes des neuromédiateurs ou modulant le relarguage des neuromédiateurs; les agents modulant la différenciation et/ou la prolifération cellulaire et/ou la pigmentation et/ou régulant la kératinisation; les agents actifs dans le traitement et/ou la prévention des cheilites; les antihistaminiques; les agents cicatrisants.

Parmi les agents ayant une certaine activité antivirale, on peut citer les agents virostatiques et/ou virucides, en particulier les agents actifs sur la classe des Papilloma virus, tels que le virus HPV1 et/ou sur le virus HPV2; on peut également citer les agents actifs sur le virus de l'Herpès Simplex de type 1 et/ou de type 2.
On peut ainsi citer, à titre d'exemple :
1/ les dérivés de la purine, en particulier de la guanine ou de l'adénine, et par exemple ceux qui sont substitués en position 9 tels que :
   . la 9-(2-hydroxyéthoxyméthyl)guanine (ou Acyclovir), ses sels ou ses esters, notamment le valérate d'acyclovir ou les esters d'aminoacides tels que valine ou isoleucine, ou de dérivés d'aminoacides tels que les esters aminométhylbenzoïque N-substitués tels que l'ester 4-morpholinobenzoïque ou l'ester 3-(ou 4-)aminométhylbenzoïque,
   . la 9-[4-hydroxy 3-(hydroxyméthyl) butyl]guanine (ou Pencyclovir), ses sels ou ses esters ou diesters,
   . la 9-β-D-Arabinofuranosyl adénine (ou Vidarabine), ses sels ou ses esters tels que l'ester 2'-acétique,
   . le 2-[2-(2-amino 9H-purine-9-yl)éthyl]-1,3-propanediol diacétate (ou Famcyclovir), ou ses sels.
2/ les dérivés de pyrimidine en particulier les dérivés de l'uracil ou de la cytosine par exemple ceux qui sont substitués en position 1 par un motif 2'-déoxyribofuranosyl tels que :
   . les alkyl-2'-déoxyuridines et notamment le vinyl -2'-déoxyuridine, le 5-éthyl -2'-déoxyuridine, et le 5-isopropyl -2'-déoxyuridine,
   . les 5-halogéno 2'-déoxyuridines et notamment le 5-iodo 2'-déoxyuridine (ou Idoxuridine), ses esters tels que l'ester 5'-éthanoïque ou 5'-benzoïque; le 5-trifluorométhyl 2'-déoxyuridine (ou Trifluridine); le 5-bromovinyl -2'-déoxyuridine;
   . le 5-iodo-2'-déoxycytidine (ou Ibacitabine).
3/ les dérivés de la guanidine tels que le 4-morpholine carboxymidoyl guanidine (ou Moroxydine), ou ses sels,
4/ les analogues pyrophosphates et leurs sels tel que l'acide phosphonoformique (ou Foscarnet),
5/ les peptides ou les protéines ou glycoprotéines modulant et/ou stimulant la réponse immunitaire et affectant la prolifération virale, tels que le pentapeptide de synthèse appelé thymopentine, ou les interférons α, β ou γ,
6/ les sels métalliques, notamment de cuivre, or, argent et/ou lithium, et en particulier les lactate ou succinate de lithium, et
7/ les oligonucléotides antisens, notamment ceux de la classe des phosphonates ou des phosphorothioates.

Parmi les agents ayant une certaine activité antibactérienne, on peut citer les agents bactériostatiques et/ou bactéricides dans le traitement topique des infections microbiennes primaires telles que l'acné, l'impétigo péribuccal chez l'enfant, ou secondaires telles que les infections intervenant lors de la phase vésiculaire de l'herpès labial.
On peut ainsi citer, à titre d'exemple :
1/ les agents de structure stéroïdienne de la famille des fusidanines, tels que l'acide fusidique et ses sels,
2/ les antibiotiques tels que ceux
   . de la famille des macrolides tels que l'érythromycine,
   . de la famille des synergistines tels que la virginiamycine,
   . de la famille des lincosamides tels que la clindamycine,
   . de la famille des aminosides tels que la néomycine, la gentamycine ou la framycétine,
   . de la famille des rifamycines tels que la rifamycine SV,
   . de type polypeptidique de la famille des polymixines tels que la polymixine B, et
   . de la classe des tétracyclines tels que l'oxytétracycline ou la minocycline.
3/ les antibactériens de la famille des quinolones tels que la ciprofloxacine, l'énoxacine, l'ofloxacine, la pefloxacine, la rosoxacine, l'acide oxolinique, l'acide nalidixique, la nadifloxacine ou la fluméquine.

Parmi les agents ayant une certaine activité anti-fongique, on peut citer l'octopirox et le cyclopirox; l'amorolfine; les imidazoles tels que l'éconazole, le miconazole, l'omoconazole, l'itraconazole, le fluconazole ou le kétoconazole; l'amphotéricine B; le tolnaftate; la griséofulvine; la terbinafine; la naphtifine.

Les agents à activité antivirale, antibactérienne ou antifongique peuvent être utilisés dans une proportion de 0,01 à 20% en poids de la composition.

Les agents ayant une certaine activité anti-inflammatoire selon l'invention peuvent avoir également une activité immunomodulatrice.
On peut citer les agents de structure stéroïdienne tels que le propionate de clobétasol, le valérate de bétaméthasone, l'hydrocortisone ou l'acéponate d'hydrocortisone, ou les agents de structure non stéroïdienne tels que le piroxicam, l'ibuprofen, l'énoxolone ou le bufexamac, ou encore l'α-bisabolol.
Ils peuvent être présents à raison de 0,01-20% en poids de la composition.

Parmi les agents antagonistes des neuromédiateurs ou modulant le relarguage des neuromédiateurs, on peut citer les antiprurigineux tels que le crotamiton; les inhibiteurs du relarguage de neuropeptides et en particulier de substance P tels que les inhibiteurs de NK1; la capsaicine et ses analogues; et les sels de lithium.
Ils peuvent être présents à raison de 0,01-20% en poids de la composition.

Parmi les agents modulant la différenciation et/ou la prolifération cellulaire et/ou la pigmentation, on peut citer les substances qui agissent par le biais des récepteurs nucléaires, tels que les récepteurs stéroïdiens, les récepteurs thyroïdiens, les récepteurs des rétinoïdes et les récepteurs de vitamine D, en particulier l'acide
6-[3-(1-adamantyl)-4-méthoxyphényl]-2-naphtoïque, la trétinoïne, l'isotrétinoïne, l'acide 9-cis rétinoïque, le calcitriol, le sécalciférol ou le calcipotriol, ou les substances agissant sur le métabolisme cellulaire telles que l'anthraline.
Parmi les agents modulant la pigmentation cutanée, on peut citer également les dépigmentants tels que l'hydroquinone, l'acide kojique ou l'acide caféïque; les agents repigmentants tels que la DHA ou les précurseurs de mélanine; les agents photosensibilisants tels que le 8-méthoxypsoralène.
Ils peuvent être présents à raison de 0,001 à 10% en poids de la composition.
Parmi les agents régulateurs de la kératinisation, on peut citer les hydroxyacides, tels que l'acide lactique, l'acide citrique ou l'acide glycolique, l'acide salicylique ou ses dérivés, ou encore l'acide 2-hydroxy,5-octanoyl benzoïque.
Ils peuvent être présents à raison de 0,1 à 30% en poids de la composition.

Parmi les agents actifs dans le traitement et/ou la prévention des cheilites, notamment d'origine climatique, mécanique ou iatrogène, on peut citer les dérivés de l'acide mandélique tels que l'acide α-acétyl mandélique.
Ils peuvent être présents à raison de 0,1-10% en poids de la composition.

Parmi les agents cicatrisants, on peut citer par exemple les vitamines A, E ou F ou leurs esters, certains peptides, les facteurs de croissance, l'allantoïne. Ils peuvent être présents à raison de 0,01 à 10% en poids de la composition. Ils permettent de prévenir, de limiter ou de traiter les fissures ou les saignements des lèvres dus au froid ou associés aux croûtes présentes sur les lèvres en phase finale de l'herpès labial.

Parmi les antihistaminiques, on peut citer la prométhazine, le méfénidramium, la triprolidine, la cinnarizine et la diphénylhydramine. Ils peuvent être présents à raison de 0,01-10% en poids de la composition.

Selon les pathologies à traiter, il peut être judicieux d'associer plusieurs agents actifs, ou encore de leur adjoindre des actifs secondaires qui vont favoriser la prévention ou le traitement de l'affection, et/ou traiter des symptômes associés à cette affection.
Parmi les agents actifs secondaires, on peut citer les anesthésiques locaux, les antiseptiques, les hydratants et/ou émollients, et les filtres solaires notamment chimiques ou minéraux.

Parmi les anesthésiques locaux, on peut citer la lidocaïne ou la tétracaïne. Ils peuvent être présents à raison de 0-10% en poids de la composition, et peuvent permettent de diminuer les sensations de brûlures/douleur ainsi que le prurit associés à certaines infections.

Parmi les antiseptiques, on peut citer des désinfectants bactériostatiques de la classe des carbanilides tels que le triclocarban; le diiséthionate d'héxamidine; les dérivés d'ammonium quaternaire tels que le chlorure de benzalkonium; le gluconate de chlorhexidine. Les antiseptiques constituent un traitement d'appoint des affections primitivement bactériennes ou susceptibles de se surinfecter; ils peuvent être présents à raison de 0-20% en poids de la composition.

Parmi les agents hydratants, lubrifiants ou émollients, on peut citer les polyols, les lipoaminoacides et certains corps gras tels que l'huile d'amande douce, le beurre de karité. Ils peuvent être présents à raison de 0 à 20% en poids de la composition.

Parmi les filtres solaires chimiques, absorbant dans les UVA et/ou UVB, on peut citer les dérivés cinnamiques tels que l'octylméthoxycinnamate, ou le 2-cyano 3,3-diphénylacrylate de 2-éthylhexyle, ou le 3,4-méthyl benzylidène 2-bornanone, ou le 4-tertbutyl 4'-méthoxydibenzoylméthane.
On peut également utiliser en tant que filtres solaires, des pigments minéraux filtrants tels que les oxydes métalliques en particulier l'oxyde de titane, éventuellement enrobé.
La composition peut comprendre 0 à 15% en poids de matière active de filtre solaire.

Ainsi, un mode de réalisation particulier de l'invention est constitué par une composition comprenant l'association d'un agent actif antiviral avec un filtre solaire chimique et/ou minéral, et en particulier un oxyde de titane.

La composition peut en outre contenir un agent favorisant la solubilisation ou la compatibilité des agents actifs avec l'excipient de la composition, et/ou favorisant la pénétration desdits agents actifs dans la peau ou les muqueuses. Il peut s'agir par exemple du myristate d'isopropyle, de l'acide oléique, de la lécithine ou de certains alcools ou glycols.

La composition peut également comprendre une phase particulaire qui peut comprendre des pigments et/ou des charges.
Les pigments peuvent être présents dans la composition à raison de 0 à 10% en poids de la composition finale. Ils peuvent être blancs ou colorés, minéraux et/ou organiques. On peut citer, parmi les pigments minéraux, les dioxydes de titane, de zirconium ou de cérium, ainsi que les oxydes de zinc, de fer ou de chrome, le bleu ferrique.
Ces pigments peuvent également être enrobés ou constituer l'enrobage notamment de particules de mica; on peut citer le mica recouvert d'oxyde de titane, d'oxyde de fer, de pigment naturel ainsi que le mica titane coloré.
Parmi les pigments organiques, on peut citer le noir de carbone, et les laques de baryum, strontium, calcium, aluminium.
Les charges, qui peuvent être présentes à raison de 0 à 30% en poids, peuvent être minérales ou de synthèse, lamellaires ou non lamellaires. On peut citer le talc, le mica, la silice, le kaolin, les poudres de Nylon et de polyéthylène, le Téflon, l'amidon le micatitane, la nacre naturelle, le nitrure de bore, les microsphères telles que l'Expancel (Nobel Industrie), le Polytrap (Dow Corning) et les microbilles de résine de silicone (Tospearls de Toshiba, par exemple).

La composition peut comprendre en outre tout additif habituellement utilisé dans le domaine considéré, tel que des antioxydants, des parfums, des huiles essentielles, des conservateurs, des vitamines, des colorants, des acides gras essentiels, des sphingocéryls, des tensioactifs, des polymères liposolubles comme les polyalkylènes, les polyacrylates et les polymères siliconés compatibles avec les corps gras. Ces additifs peuvent être présents dans la composition à raison de 0-10% en poids.

Bien entendu, l'homme du métier veillera à choisir tous les éventuels composés complémentaires, c'est-à-dire les actifs secondaires, l'agent favorisant la solubilisation, les composés de la phase particulaire et les additifs, ainsi que leur quantité dans la composition, de manière telle que, d'une part, les propriétés avantageuses de la composition selon l'invention ne soient pas, ou substantiellement pas, altérées par l'adjonction envisagée, et d'autre part, que leur adjonction reste compatible avec la nature de ladite composition et avec son utilisation par voie topique.

Les compositions selon l'invention peuvent se présenter sous la forme d'un produit pour le soin ou le traitement de la peau et des muqueuses.
Elles peuvent en particulier se présenter sous la forme d'une composition liquide ou fluide, huileuse ou gélifiée, d'une pâte souple ou d'une pâte dure et coulée telle qu'un bâton ou stick.
Par pâte souple, on entend une pâte dont on peut mesurer la viscosité, par opposition à la structure solide d'un bâton, ou stick, dont on ne peut pas mesurer la viscosité. Ladite viscosité dynamique à 25°C est généralement comprise entre 3 et 35 Pa.s, mesurée avec un viscosimètre rotatif CONTRAVES TV équipé d'un mobile "MS-r4" à la fréquence de 60 Hz.

Les compositions selon l'invention peuvent être préparées selon des procédés qui ne différent en rien des procédés classiquement utilisés notamment en cosmétique ou en pharmacie topique, et parfaitement connus de l'homme de l'art. Ces procédés consistent dans le mélange des différents constituants de la composition, préalablement chauffés généralement à 95-100°C lorsque des cires sont présentes, puis à leur éventuel coulage selon la forme souhaitée avant refroidissement.
Certains constituants de la composition peuvent être introduits à une température inférieure, en particulier lorsqu'ils sont susceptibles de réagir à la chaleur. Toutefois, il n'est généralement pas possible de les introduire à froid sans observer de cristallisation et/ou de prise en masse au moins partielle des cires présentes.

Dans ce cas, et en particulier lorsque des cires sont présentes dans la composition, il est possible de la préparer grâce à un procédé mettant en oeuvre au moins un mélangeur-extrudeur.
Selon ce procédé, on peut préparer tout d'abord un prémélange comprenant au moins une partie des différents constituants de la composition, dont les cires éventuelles; on peut chauffer ce prémélange en le malaxant, à une température à laquelle il fond; on peut alors ajouter à une température adéquate, pouvant être de l'ordre de la température ambiante, en une ou plusieurs fois, le reste des constituants tout en continuant le malaxage; puis malaxer le mélange obtenu pendant au moins une partie de son refroidissement jusqu'à température ambiante s'il était à une température supérieure.
L'opération de malaxage est effectuée au moins partiellement dans un extrudeur. L'huile volatile est de préférence ajoutée à la fin du procédé d'extrusion, à température ambiante. Toutefois, l'huile volatile peut également être ajoutée pendant l'étape de refroidissement, de préférence à une température inférieure ou égale à 45°C environ.

Ce procédé permet d'obtenir une composition se présentant sous forme de pâte souple, qui est homogène et dans laquelle tous les constituants sont mélangés de manière adéquate, sans avoir à chauffer les éventuels constituants réactifs à la chaleur à une température trop importante.
On peut effectuer l'opération de chauffage selon toute technique connue.
Dans un mode particulier de réalisation de l'invention, les opérations de chauffage et de malaxage, voire de refroidissement, sont réalisées en totalité dans un ou plusieurs extrudeurs disposés à la suite les uns des autres, et de préférence dans un extrudeur bivis unique.
De plus, il est possible, en adaptant la filière de sortie du mélangeur-extrudeur, de conditionner la composition en ligne à la sortie dudit mélangeur-extrudeur.
Les conditions dans lesquelles l'extrusion peut être effectuée sont décrites dans la demande de brevet FR94-00756 dont le contenu est incorporé à la présente demande par référence.

L'invention a également pour objet un procédé de traitement dermatologique ou pharmaceutique de la peau et/ou des muqueuses, consistant à appliquer une composition telle que ci-dessus définie.

L'invention est illustrée plus en détail dans les exemples suivants.

### Exemple 1

On prépare un stick pour les lèvres ayant la composition suivante:

| *phase grasse* | |
|---|---|
| . cyclopentadiméthylsiloxane | 42 g |
| . polyphénylméthylsiloxane (DC556 Fluid de Dow Corning) | 25 g |
| . cires de silicone | 10 g |
| . cires hydrocarbonées (notamment polyéthylène) | 10 g |

| *phase pulvérulente* | |
|---|---|
| . dioxyde de titane | 5 g |
| . charges (poudre de Nylon, amidon) | 2 g |

| *actifs* | |
|---|---|
| . Idoxuridine | 1 g |
| . 3,4-méthyl benzylidène bornanone | 3 g |
| . 4-tertbutyl 4'-méthoxydibenzoylméthane | 2 g |

On prépare la composition de manière usuelle, en chauffant les corps gras, sauf les huiles volatiles, à 95-100°C et en les mélangeant.
On ajoute ensuite le dioxyde de titane et les charges. On mélange le tout à l'aide d'une turbine Moritz à la vitesse de 3000 tr/min. On ajoute les huiles volatiles et les actifs préalablement dispersés dans une aliquote de phase huileuse juste avant le coulage. On peut alors couler ledit mélange dans des moules adéquats.

On obtient ainsi un stick pour les lèvres, de texture agréable, qui s'étale bien et s'applique uniformément. Le film est confortable à porter dans le temps et ne migre pas.
Ce stick peut être indiqué dans la prévention de l'herpès labial récurent, notamment en application quotidienne, dans le but de diminuer la fréquence des récidives et la durée des poussées d'herpès.

### Exemple 2

On prépare une pâte souple ayant la composition suivante:

| | |
|---|---|
| . cyclopentadiméthylsiloxane | 45 g |
| . polyphénylméthylsiloxane (DC556 Fluid de Dow Corning) | 25 g |
| . cire de silicone | 10 g |
| . cire de polyéthylène | 5 g |
| . alkyl diméthicone | 5 g |
| . dioxyde de titane | 5 g |
| . charges (notamment poudre de Nylon) | 3 g |
| . acide fusidique anhydre | 2 g |

On prépare la composition de manière usuelle, en chauffant les corps gras, sauf les huiles volatiles, à 95-100°C et en les mélangeant. On ajoute ensuite le dioxyde de titane et les charges. On mélange le tout à l'aide d'une turbine Moritz à la vitesse de 3000 tr/min.
On ajoute les huiles volatiles et l'acide fusidique préalablement dispersé dans une aliquote de phase huileuse juste avant le coulage. On peut alors couler ledit mélange dans des conditionnements adéquats.

On obtient ainsi une pâte souple, de texture agréable, qui s'étale bien et s'applique uniformément. Le film est confortable à porter dans le temps et ne migre pas.
Cette composition peut être utilisée dans le traitement des infections bactériennes atteignant le visage, telles que l'impétigo péribuccal chez l'enfant.

## Revendications

1. Composition dermatologique ou pharmaceutique à application topique, comprenant une huile volatile, une huile siliconée phénylée et au moins un agent actif dermatologiquement et/ou pharmaceutiquement.

2. Composition selon la revendication 1, dans laquelle l'huile volatile est choisie parmi les huiles hydrocarbonées ou les huiles siliconées, cycliques et linéaires, seules ou en mélange.

3. Composition selon l'une des revendications précédentes, dans laquelle l'huile volatile est choisie parmi la cyclotétradiméthylsiloxane, la cyclopentadiméthylsiloxane, la cyclohexadiméthylsiloxane, le méthylhexyldiméthylsiloxane, les isoparaffines et leurs mélanges.

4. Composition selon l'une des revendications précédentes, dans laquelle l'huile volatile est présente à raison de 8 à 80% en poids, de préférence 30 à 60%, par rapport au poids de la composition.

5. Composition selon l'une des revendications précédentes, dans laquelle l'huile siliconée phénylée est choisie parmi les huiles de formule (I) et leurs mélanges : 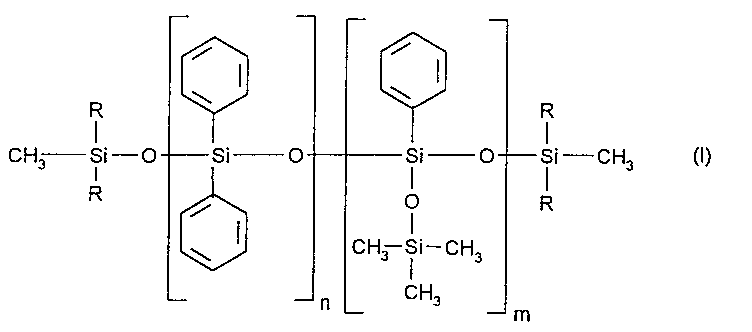 dans laquelle
. R est un radical alkyle en C1-C30, un radical aryle ou un radical aralkyle,
. n est un nombre entier compris entre 0 et 100,
. m est un nombre entier compris entre 0 et 100, sous réserve que la somme m+n est comprise entre 1 et 100.

6. Composition selon l'une des revendications précédentes, dans laquelle l'huile siliconée phénylée est présente à raison de 1 à 35% en poids, de préférence 20 à 30%, par rapport au poids de la composition.

7. Composition selon l'une des revendications précédentes, dans laquelle l'agent actif est choisi parmi les agents actifs contre les micro-organismes, notamment à activité antivirale, antibactérienne ou antifongique; les agents à activité anti-inflammatoire ou immunomodulatrice; les agents antagonistes des neuromédiateurs ou modulant le relarguage des neuromédiateurs; les agents modulant la différenciation et/ou la prolifération cellulaire et/ou la pigmentation et/ou régulant la kératinisation; les agents actifs dans le traitement et/ou la prévention des cheilites; les antihistaminiques; les agents cicatrisants.

8. Composition selon la revendication 7, dans laquelle l'agent actif à activité antivirale est choisi parmi les agents actifs sur la classe des Papilloma virus; les agents actifs sur le virus de l'Herpès Simplex de type 1 et/ou 2; les dérivés de purine; les dérivés de pyrimidine; les dérivés de guanidine; les analogues pyrophosphates et leurs sels; les peptides ou les protéines ou glycoprotéines modulant et/ou stimulant la réponse immunitaire et affectant la prolifération virale; les sels métalliques, notamment de cuivre, or, argent et/ou lithium; les oligonucléotides antisens.

9. Composition selon la revendication 7, dans laquelle l'agent actif à activité antibactérienne est choisi parmi les agents de structure stéroïdienne de la famille des fusidanines; les antibiotiques tels que ceux de la famille des macrolides, des synergistines, des lincosamides, des aminosides, des rifamycines, des polymixines, des tétracyclines; les antibactériens de la famille des quinolones.

10. Composition selon la revendication 7, dans laquelle l'agent actif à activité antifongique est choisi parmi l'octopirox, le cyclopirox, l'amorolfine, les imidazoles, l'amphotéricine B, le tolnaftate, la griséofulvine, la terbinafine et la naphtifine.

11. Composition selon la revendication 7, dans laquelle l'agent actif à activité anti-inflammatoire est choisi parmi les agents de structure stéroïdienne tels que le propionate de clobétasol, le valérate de bétaméthasone, l'hydrocortisone ou l'acéponate d'hydrocortisone; les agents de structure non stéroïdienne tels que le piroxicam, l'ibuprofen, l'énoxolone ou le bufexamac; l'α-bisabolol.

12. Composition selon la revendication 7, dans laquelle l'agent antagoniste des neuromédiateurs ou modulant le relarguage des neuromédiateurs, est choisi parmi les antiprurigineux tels que le crotamiton; les inhibiteurs du relarguage de neuropeptides et en particulier de substance P tels que les inhibiteurs de NK1; la capsaicine et ses analogues; et les sels de lithium.

13. Composition selon l'une des revendications 7 à 12, dans laquelle l'agent actif contre les micro-organismes, l'agent à activité anti-inflammatoire ou l'agent antagoniste des neuromédiateurs ou modulant le relarguage des neuromédiateurs, est présent à raison de 0,01-20% en poids de la composition.

14. Composition selon la revendication 7, dans laquelle l'agent modulant la différenciation et/ou la prolifération cellulaire et/ou la pigmentation est choisi parmi les substances agissant par le biais des récepteurs nucléaires tels que les récepteurs stéroïdiens, les récepteurs thyroïdiens, les récepteurs des rétinoïdes, les récepteurs de vitamine D; les substances agissant sur le métabolisme cellulaire, les dépigmentants, les agents repigmentants; les agents photosensibilisants.

15. Composition selon l'une des revendications 7 ou 14, dans laquelle l'agent modulant est présent à raison de 0,001-10% en poids de la composition.

16. Composition selon la revendication 7, dans laquelle l'agent régulateur de la kératinisation est choisi parmi les hydroxyacides.

17. Composition selon l'une des revendications 7 ou 16, dans laquelle l'agent régulateur de la kératinisation est présent à raison de 0,1-30% en poids de la composition.

18. Composition selon la revendication 7, dans laquelle l'agent cicatrisant est choisi parmi les vitamines A, E ou F ou leurs esters, les facteurs de croissance, l'allantoïne.

19. Composition selon la revendication 7, dans laquelle l'agent antihistaminique est choisi parmi la prométhazine, le méfénidramium, la triprolidine, la cinnarizine et la diphénylhydramine.

20. Composition selon l'une des revendications 7, 18 ou 19, dans laquelle l'agent cicatrisant ou l'agent antihistaminique est présent à raison de 0,01-10% en poids de la composition.

21. Composition selon la revendication 7, dans laquelle l'agent actif dans le traitement et/ou la prévention des cheilites est choisi parmi les dérivés de l'acide mandélique tels que l'acide α-acétyl mandélique.

22. Composition selon l'une des revendications 7 ou 21, dans laquelle l'agent actif dans le traitement et/ou la prévention des cheilites est présent à raison de 0,1-10% en poids de la composition.

23. Composition selon l'une des revendications précédentes, comprenant en outre au moins un agent actif secondaire choisi parmi les anesthésiques locaux, les antiseptiques, les hydratants et/ou émollients, et/ou les filtres solaires chimiques ou minéraux.

24. Composition selon la revendication 23 comprenant l'association d'un agent actif antiviral et d'un filtre solaire chimique et/ou minéral, en particulier un oxyde de titane.

25. Composition selon l'une des revendications précédentes, comprenant en outre un agent favorisant la solubilisation ou la compatibilité des agents actifs avec la composition, et/ou favorisant la pénétration desdits agents actifs dans la peau ou les muqueuses.

26. Composition selon l'une des revendications précédentes, se présentant sous la forme d'une composition liquide ou fluide, huileuse ou gélifiée, d'une pâte souple ou d'une pâte dure et coulée telle qu'un bâton ou stick, susceptible d'être utilisée en tant que produit pour le soin ou le traitement de la peau et des muqueuses.

27. Procédé de préparation d'une composition selon l'une quelconques des revendications précédentes, dans lequel on prépare tout d'abord un prémélange comprenant au moins une partie des différents constituants de la composition, dont les cires éventuelles; on chauffe ce prémélange en le malaxant, à une température à laquelle il fond; on ajoute à une température adéquate, pouvant être de l'ordre de la température ambiante, le reste des constituants tout en continuant le malaxage; puis on malaxe le mélange obtenu pendant au moins une partie de son refroidissement jusqu'à température ambiante s'il était à une température supérieure, caractérisé par le fait que l'opération de malaxage est effectuée au moins partiellement dans un extrudeur.

28. Procédé selon la revendication 27 dans lequel les opérations de chauffage et de malaxage, voire de refroidissement, sont réalisées en totalité dans un ou plusieurs extrudeurs disposés à la suite les uns des autres.

29. Procédé selon la revendication 27 dans lequel les opérations de chauffage, de malaxage et de refroidissement, sont réalisées dans un extrudeur bi-vis unique.

30. Utilisation de l'association d'une huile volatile et d'une huile siliconée phénylée, pour la preperation d'une composition dermatologique ou pharmaceutique à application topique comprenant au moins un agent actif dermatologiquement et/ou pharmaceutiquement, dans le but d'améliorer le transfert et/ou la migration de ladite composition.

## Claims

1. Dermatological or pharmaceutical composition for topical application comprising a volatile oil, a phenyl-containing silicone oil and at least one dermatologically and/or pharmaceutically active agent.

2. Composition according to Claim 1, in which the volatile oil is chosen from cyclic and linear silicone oils or hydrocarbon oils, alone or as a mixture.

3. Composition according to either of the preceding claims, in which the volatile oil is chosen from cyclotetradimethylsiloxane, cyclopentadimethylsiloxane, cyclohexadimethylsiloxane, methylhexyldimethylsiloxane, isoparaffins and their mixtures.

4. Composition according to one of the preceding claims, in which the volatile oil is present in the proportion of 8 to 80 % by weight, preferably 30 to 60 %, with respect to the weight of the composition.

5. Composition according to one of the preceding claims, in which the phenyl-containing silicone oil is chosen from the oils of formula (I) and their mixtures: 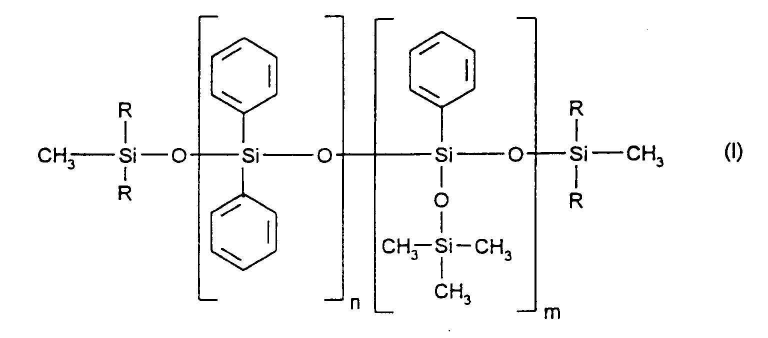 in which
• R is a C₁-C₃₀ alkyl radical, an aryl radical or an aralkyl radical,
• n is an integer between 0 and 100,
• m is an integer between 0 and 100, with the proviso that the sum m+n is between 1 and 100.

6. Composition according to one of the preceding claims, in which the phenyl-containing silicone oil is present in the proportion of 1 to 35 % by weight, preferably 20 to 30 %, with respect to the weight of the composition.

7. Composition according to one of the preceding claims, in which the active agent is chosen from agents which are active against microorganisms, in particular with an antiviral, antibacterial or antifungal activity; agents with an antiinflammatory or immunomodulating activity; agents which are antagonists of neuromediators or which modulate the release of neuromediators; agents which modulate cell differentiation and/or cell proliferation and/or pigmentation and/or which regulate keratinization; agents which are active in the treatment and/or the prevention of cheilites; antihistamines; or healing agents.

8. Composition according to Claim 7, in which the active agent with antiviral activity is chosen from agents which are active with respect to the class of the Papillomaviruses; agents which are active with respect to the type 1 and/or 2 herpes simplex virus; purine derivatives; pyrimidine derivatives; guanidine derivatives; pyrophosphate analogues and their salts; peptides or proteins or glycoproteins which modulate and/or stimulate the immune response and which affect viral proliferation; metal salts, in particular of copper, gold, silver and/or lithium; or antisense oligonucleotides.

9. Composition according to Claim 7, in which the active agent with antibacterial activity is chosen from agents of steroidal structure from the family of the fusidanines; antibiotics, such as those from the family of the macrolides, of the synergistins, of the lincosamides, of the aminosides, of the rifamycins, of the polymyxins or of the tetracyclines; or antibacterials from the family of the quinolones.

10. Composition according to Claim 7, in which the active agent with antifungal activity is chosen from octopirox, cyclopirox, amorolfine, the imidazoles, amphotericin B, tolnaftate, griseofulvin, terbinafine and naftifine.

11. Composition according to Claim 7, in which the active agent with antiinflammatory activity is chosen from agents of steroidal structure, such as clobetasol propionate, betamethasone valerate, hydrocortisone or hydrocortisone aceponate; agents of non-steroidal structure, such as piroxicam, ibuprofen, enoxolone or bufexamac; or α-bisabolol.

12. Composition according to Claim 7, in which the agent which is an antagonist of neuromediators or which modulates the release of neuromediators is chosen from antipruritics, such as crotamiton; inhibitors of the release of neuropeptides and in particular of substance P, such as inhibitors of NK1; capsaicin and its analogues; and lithium salts.

13. Composition according to one of Claims 7 to 12, in which the agent which is active against microorganisms, the agent with antiinflammatory activity or the agent which is an antagonist of neuromediators or which modulates the release of neuromediators is present in the proportion of 0.01-20 % by weight of the composition.

14. Composition according to Claim 7, in which the agent which modulates cell differentiation and/or cell proliferation and/or pigmentation is chosen from substances which act by means of nuclear receptors, such as steroidal receptors, thyroidal receptors, receptors for retinoids or receptors for vitamin D; substances which act on cell metabolism, depigmenting agents or repigmenting agents; or photosensitizing agents.

15. Composition according to either of Claims 7 and 14, in which the modulating agent is present in the proportion of 0.001-10 % by weight of the composition.

16. Composition according to Claim 7, in which the agent for regulating keratinization is chosen from hydroxy acids.

17. Composition according to either of Claims 7 and 16, in which the agent for regulating keratinization is present in the proportion of 0.1-30 % by weight of the composition.

18. Composition according to Claim 7, in which the healing agent is chosen from vitamins A, E or F or their esters, growth factors or allantoin.

19. Composition according to Claim 7, in which the antihistamine agent is chosen from promethazine, mefenidramium, triprolidine, cinnarizine and diphenylhydramine.

20. Composition according to one of Claims 7, 18 and 19, in which the healing agent or the antihistamine agent is present in the proportion of 0.01-10 % by weight of the composition.

21. Composition according to Claim 7, in which the agent which is active in the treatment and/or the prevention of cheilites is chosen from derivatives of mandelic acid, such as α-acetylmandelic acid.

22. Composition according to either of Claims 7 and 21, in which the agent which is active in the treatment and/or the prevention of cheilites is present in the proportion of 0.1-10 % by weight of the composition.

23. Composition according to one of the preceding claims comprising, in addition, at least one supplementary active agent chosen from local anaesthetics, antiseptics, moisturizers and/or emollients, and/or chemical or inorganic sunscreening agents.

24. Composition according to Claim 23, comprising the combination of an antiviral active agent and of a chemical and/or inorganic sunscreening agent, in particular a titanium oxide.

25. Composition according to one of the preceding claims comprising, in addition, an agent which promotes the solubilization or the compatibility of the active agents with the composition and/or which promotes the penetration of the said active agents into the skin or the mucous membranes.

26. Composition according to one of the preceding claims, which is provided in the form of a liquid or fluid, oily or gelled composition, of a supple paste or of a hard and cast paste, such as a stick, capable of being used as a product for caring for or treating the skin and mucous membranes.

27. Process for the preparation of a composition according to any one of the preceding claims, in which, first of all, a premix is prepared comprising at least a part of the various constituents of the composition, including the possible waxes; this premix is heated, while mixing it, to a temperature at which it melts; the remainder of the constituents are added at a suitable temperature, which can be of the order of room temperature, while continuing the mixing; the mixture obtained is then mixed during at least a part of its cooling to room temperature, if it was at a higher temperature, characterized in that the mixing operation is carried out at least partially in an extruder.

28. Process according to Claim 27, in which the heating and mixing, indeed cooling, operations are carried out entirely in one or a number of extruders arranged in series with respect to one another.

29. Process according to Claim 27, in which the heating, mixing and cooling operations are carried out in a single twin-screw extruder.

30. Use of the combination of a volatile oil and of a phenyl-containing silicone oil for the preparation of a dermatological or pharmaceutical composition for topical application comprising at least one dermatologically and/or pharmaceutically active agent, with the aim of improving the transfer and/or the migration of the said composition.

## Patentansprüche

1. Dermatologische oder pharmazeutische Zusammensetzung zur topischen Anwendung, enthaltend ein flüchtiges Öl, ein phenyliertes Siliconöl und mindestens einen dermatologischen und/oder pharmazeutischen Wirkstoff.

2. Zusammensetzung nach Anspruch 1, wobei das flüchtige Öl unter cyclischen und linearen Kohlenwasserstoffölen oder Siliconölen, die einzeln oder im Gemisch vorhanden sind, ausgewählt ist.

3. Zusammensetzung nach einem der vorstehenden Ansprüche, wobei das flüchtige Öl unter Cyclotetradimethylsiloxan, Cyclopentadimethylsiloxan, Cyclohexadimethylsiloxan, Methylhexyldimethylsiloxan, Isoparaffinen und deren Gemischen ausgewählt ist.

4. Zusammensetzung nach einem der vorstehenden Ansprüche, wobei das flüchtige Öl in einem Anteil von 8 bis 80 Gew.-% und vorzugsweise von 30-bis 60 Gew.-%, bezogen auf das Gewicht der Zusammensetzung, vorhanden ist.

5. Zusammensetzung nach einem der vorstehenden Ansprüche, wobei das phenylierte Siliconöl unter Ölen der Formel (I) und deren Gemischen ausgewählt ist: 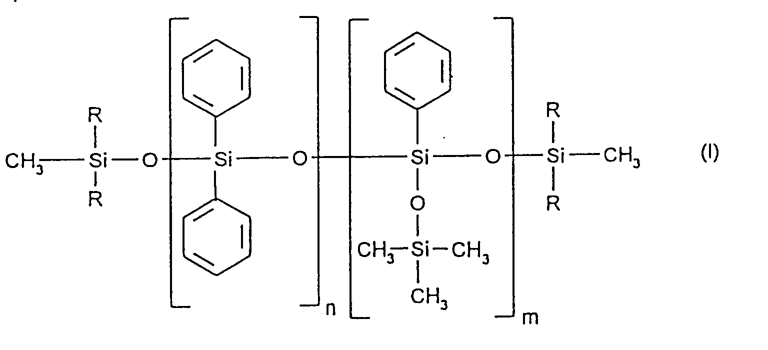 worin
- R einen C₁-C₃₀-Alkylrest, einen Arylrest oder einen Aralkylrest bedeutet,
- n eine ganze Zahl von 0 bis 100 bedeutet,
- m eine ganze Zahl von 0 bis 100 bedeutet, mit der Maßgabe, daß die Summe m + n im Bereich von 1 bis 100 liegt.

6. Zusammensetzung nach einem der vorstehenden Ansprüche, wobei das phenylierte Siliconöl in einem Anteil von 1 bis 35 Gew.-% und vorzugsweise von 20 bis 30 Gew.-%, bezogen auf das Gewicht der Zusammensetzung, vorliegt.

7. Zusammensetzung nach einem der vorstehenden Ansprüche, wobei der Wirkstoff aus folgender Gruppe ausgewählt ist: Wirkstoffe gegen Mikroorganismen, insbesondere mit antiviraler, antibakterieller oder antimykotischer Wirkung; Wirkstoffe mit entzündungshemmender oder immunomodulierender Wirkung; Mittel mit antagonistischer Wirkung gegen Neuromediatoren oder modulierender Wirkung bezüglich der Ausschüttung von Neuromediatoren; Mittel zur Modulierung der Differenzierung und/oder Vermehrung von Zellen und/oder Pigmentierung und/oder Regulierung der Keratinisierung; Wirkstoffe zur Therapie und/oder Prophylaxe von Cheilitis; Antihistaminika; und wundheilende Mittel.

8. Zusammensetzung nach Anspruch 7, wobei der Wirkstoff mit antiviraler Wirkung ausgewählt ist aus der Gruppe: Wirkstoffe gegen die Klasse der Papilloma-Viren; Wirkstoffe gegen den Virus von Herpes simplex Typ 1 und/oder 2; Purinderivate; Pyrimidinderivate; Guanidinderivate; Pyrophosphat-Analoge und deren Salze; Peptide oder Proteine oder Glycoproteine, die die Immunantwort modulieren und/oder stimulieren und die virale Vermehrung beeinflussen; Metallsalze, insbesondere von Kupfer oder Silber und/oder Lithium; und antisense-Oligonucleotide.

9. Zusammensetzung nach Anspruch 7, in der der Wirkstoff mit antibakterieller Wirkung ausgewählt ist aus der Gruppe: Mittel mit Steroidstruktur aus der Familie der Fusidanine; Antibiotika, beispielsweise aus der Familie der Macrolide, Synergistine, Lincosamide, Aminoside, Rifamycine, Polymixine und Tetracycline; und antibakterielle Mittel aus der Familie der Chinolone.

10. Zusammensetzung nach Anspruch 7, wobei der Wirkstoff mit antimykotischer Wirkung ausgewählt ist unter: Octopirox, Cyclopirox, Amorolfin, Imidazole, Amphotericin B, Tolnaftat, Griseofulvin, Terbinafin und Naphtifin.

11. Zusammensetzung nach Anspruch 7, wobei der Wirkstoff mit entzündungshemmender Wirkung ausgewählt ist aus der Gruppe: Mittel mit Steroidstruktur, wie Clobetasolpropionat, Betamethason-valerat, Hydrocortison oder Hydrocortison-aceponat; Mittel mit Nichtsteroidstruktur, wie Piroxicam, Ibuprofen, Enoxolon oder Bufexamac; und α-Bisabolol.

12. Zusammensetzung nach Anspruch 7, wobei das antagonistische Mittel gegen Neuromediatoren oder zur Modulation der Ausschüttung von Neuromediatoren ausgewählt ist aus folgender Gruppe: juckreizhemmende Mittel, wie Crotamiton; Inhibitoren der Ausschüttung von Neuropeptiden und insbesondere der Substanz P, wie die Inhibitoren von NK1; Capsaicin und dessen Analoge; und Lithiumsalze.

13. Zusammensetzung nach einem der Ansprüche 7 bis 12, wobei der Wirkstoff gegen Mikroorganismen, das Mittel mit entzündungshemmender Wirkung oder das antagonistische Mittel gegen Neuromediatoren oder zur Modulation der Aussschüttung von Neuromediatoren in einem Anteil von 0,01 bis 20 Gew.-% der Zusammensetzung vorhanden ist.

14. Zusammensetzung nach Anspruch 7, wobei das Mittel zur Modulation der Differenzierung und/oder Vermehrung von Zellen und/oder der Pigmentierung aus folgender Gruppe ausgewählt ist: Substanzen, die durch den "Bias" von Nuclearrezeptoren, wie Steroidrezeptoren, Thyroidrezeptoren, Retinoidrezeptoren und Vitamin D-Rezeptoren, wirken; Substanzen, die auf den Zellstoffwechsel wirken; Depigmentierungsmittel; Pigmentierungsmittel; und photosensibilisierende Mittel.

15. Zusammensetzung nach einem der Ansprüche 7 oder 14, wobei das modulierende Mittel in einem Anteil von 0,001 bis 10 Gew.-% der Zusammensetzung vorhanden ist.

16. Zusammensetzung nach Anspruch 7, wobei das Mittel zur Regulierung der Keratinisierung unter Hydroxysäuren ausgewählt ist.

17. Zusammensetzung nach einem der Ansprüche 7 oder 16, wobei das Mittel zur Regulierung der Keratinisierung in einem Anteil von 0,1 bis 30 Gew.-% der Zusammensetzung enthalten ist.

18. Zusammensetzung nach Anspruch 7, in der das Wundheilungsmittel unter den Vitaminen A, E, F und deren Estern, den Wachstumsfaktoren und Allantoin ausgewählt ist.

19. Zusammensetzung nach Anspruch 7, wobei das Antihistaminikum unter Promethazin, Mefenidramium, Triprolidin, Cinnarizin und Diphenylhydramin ausgewählt ist.

20. Zusammensetzung nach einem der Ansprüche 7, 18 oder 19, wobei das wundheilende Mittel oder das Antihistaminikum in einem Anteil von 0,01 bis 10 Gew.-% der Zusammensetzung vorhanden sind.

21. Zusammensetzung nach Anspruch 7, wobei der Wirkstoff zur Therapie und/oder Prophylaxe von Cheilitis unter Mandelsäurederivaten, wie α-Acetylmandelsäure, ausgewählt ist.

22. Zusammensetzung nach einem der Ansprüche 7 oder 21, wobei der Wirkstoff zur Behandlung und/oder Prophylaxe von Cheilitis in einem Anteil von 0,1 bis 10 Gew.-% der Zusammensetzung vorhanden ist.

23. Zusammensetzung nach einem der vorstehenden Ansprüche, umfassend mindestens einen sekundären Wirkstoff, der unter Lokalanästhetika, antiseptischen Mitteln, hydratisierenden Mitteln und/oder Emollentien und/oder chemischen oder mineralischen Sonnenschutzfiltern ausgewählt ist.

24. Zusammensetzung nach Anspruch 23, umfassend eine Kombination aus einem antiviralen Wirkstoff und einem chemischen und/oder mineralischen Sonnenschutzfilter, insbesondere Titanoxid.

25. Zusammensetzung nach einem der vorstehenden Ansprüche, umfassend ein Mittel zur Begünstigung der Solubilisierung und/oder Verträglichkeit von Wirkstoffen mit der Zusammensetzung und/oder zur Begünstigung der Penetration dieser Wirkstoffe in die Haut oder die Schleimhäute.

26. Zusammensetzung nach einem der vorstehenden Ansprüche, in Form einer flüssigen oder fluiden, öligen oder gelierten Zusammensetzung, einer weichen Paste oder einer harten und gegossenen Paste, wie eines Stabs oder Stifts, geeignet zur Verwendung als Produkt zur Pflege oder Behandlung der Haut oder der Schleimhäute.

27. Verfahren zur Herstellung einer Zusammensetzung nach einem der vorstehenden Ansprüche, wobei man zunächst ein Vorgemisch herstellt, das mindestens einen Teil der verschiedenen Bestandteile der Zusammensetzung enthält, darunter die gegebenenfalls vorhandenen Wachse; das Vorgemisch unter Kneten auf eine Temperatur erwärmt, bei der es schmilzt; bei einer geeigneten Temperatur, die in der Größenordnung von Raumtemperatur liegen kann, den Rest der Bestandteile unter fortgesetztem Kneten zusetzt; schließlich das erhaltene Gemisch während mindestens eines Teils seiner Abkühlung auf Umgebungstemperatur, sofern es sich bei einer höheren Temperatur befand, knetet, dadurch gekennzeichnet, daß der Knetvorgang mindestens teilweise in einem Extruder durchgeführt wird.

28. Verfahren nach Anspruch 27, wobei die Vorgänge des Erwärmens und des Knetens und sogar der Abkühlung insgesamt in einem oder mehreren Extrudern, die nacheinander angeordnet sind, durchgeführt werden.

29. Verfahren nach Anspruch 27, wobei die Vorgänge des Erwärmens, des Knetens und des Abkühlens in einem einzigen Doppelschneckenextruder durchgeführt werden.

30. Verwendung der Kombination aus einem flüchtigen Öl und einem phenylierten Siliconöl zur Herstellung einer dermatologischen oder pharmazeutischen Zusammensetzung zur topischen Anwendung, die mindestens einen dermatologischen und/oder pharmazeutischen Wirkstoff enthält, mit dem Ziel, die Übertragung und/oder Wanderung dieser Zusammensetzung zu verbessern.
